# EUROPEAN PATENT APPLICATION

(11) **EP 4 455 304 A1**
(43) Date of publication of application: **30.10.2024**
(21) Application number: 23315136.4
(22) Date of filing: 28.04.2023
(51) Int. Cl.: C12Q 1/6848, G16B 25/20, G16B 25/10

(54) **NUCLEIC ACID AMPLIFICATION PROCESS CONTROLS**

(71) Applicant: Mobidiag Oy, 02150 Espoo (FI)
(72) Inventor: Hennetin, Jérôme, 75011 PARIS (FR); Bondet, Jean-Sebastien, 75011 PARIS (FR)
(74) Representative: Regimbeau

(57) **Abstract**

Disclosed are methods, systems, computer readable media for monitoring 1 monitoring fluorescence signals from a nucleic acid amplification reaction for abnormal fluorescence detection. Exemplary methods include determining fluorescence-based values from fluorophore-containing nucleic acid amplification reaction mixtures and determining whether those values satisfy predetermined thresholds and/or ranges or otherwise indicate abnormalities.

## Description

### FIELD

This disclosure relates to the field of nucleic acid amplification. More particularly, the disclosure concerns methods, materials, apparatuses, and systems for monitoring fluorescence signals from a nucleic acid amplification reaction for abnormal fluorescence detection.

### INTRODUCTION

Nucleic acid amplification reactions are widely used in research and clinical laboratories for detecting genetic disorders and infectious diseases. Nucleic amplification reactions utilize enzymes such as polymerases or ligases, separately or in combination, to generate multiple copies of a target nucleic acid sequence in primer extension reactions which incorporate nucleotides or by ligations of adjacent probes that are complementary to a target nucleic acid sequence. In such reactions, each template generates more copies, and the copies may themselves become templates. The nucleic acid copies are referred to as "amplicons." Production of amplicons can be tracked in real time using fluorescence. For example, a probe oligonucleotide containing a quencher and a fluorophore can be configured to undergo degradation, as in a TaqMan^{®} assay, or a conformational change, as with a molecular torch, in an amplicon-dependent manner; the degradation or conformational change reduces or eliminates quenching of the fluorophore and thereby increases fluorescence, which serves as a readout of amplicon production.

Fluorescence-based detection methods require excitation of the fluorophore and detection of emitted fluorescent light. Failure of either the light source responsible for excitation or the fluorescence detector responsible for detecting the emitted light can arise during nucleic acid amplification. Such abnormalities can lead to unreliable or inconsistent results, such as false negative results, and wasted reagents. Existing amplification assays may not adequately control for such abnormalities and modes of failure. Accordingly, there is a need for improved nucleic acid amplification process controls.

Disclosed herein are materials, methods, and systems that relate to detecting abnormalities in fluorescence detection. The methods disclosed herein can detect abnormal fluorescence detection, e.g., resulting from equipment failure, that may occur during nucleic acid amplification. The present disclosure is based in part on the finding that fluorescence intensities measured over time from a nucleic acid amplification reaction do not fall below a threshold level when fluorescence detection is occurring normally. Accordingly, a predetermined threshold can be set and fluorescence intensities measured from a nucleic acid amplification reaction can be compared to the predetermined threshold. A fluorescence intensity measured from the reaction below the threshold results in detection of abnormal fluorescence detection.

### SUMMARY

The following embodiments are among those provided by the disclosure.

Embodiment 1 is a method of monitoring fluorescence signals from a nucleic acid amplification reaction for abnormal fluorescence detection, the method comprising:
a) measuring fluorescence signal from the nucleic acid amplification reaction at a plurality of time points in a time period during which the nucleic acid amplification reaction is occurring;
b) determining a plurality of fluorescence intensities at at least a portion of the plurality of time points from the measured fluorescence signals;
c) comparing each of the plurality of fluorescence intensities to a predetermined threshold, wherein abnormal fluorescence detection is detected if a fluorescence intensity is below the predetermined threshold.

Embodiment 2 is a nucleic acid amplification system comprising: a docking station configured to receive a vessel comprising at least one nucleic acid amplification reaction chamber; a fluorescence detector configured to measure fluorescence from a nucleic acid amplification reaction in the nucleic acid reaction chamber; and a processor operably linked to the fluorescence detector and a memory;
the memory comprising instructions that when executed by the processor cause the nucleic acid amplification system to perform a method of monitoring fluorescence signals from a nucleic acid amplification reaction for abnormal fluorescence detection, the method comprising:
a) measuring fluorescence signal from the nucleic acid amplification reaction at a plurality of time points in a time period during which the nucleic acid amplification reaction is occurring;
b) determining a plurality of fluorescence intensities at at least a portion of the plurality of time points from the measured fluorescence signals;
c) comparing each of the plurality of fluorescence intensities to a predetermined threshold, wherein abnormal fluorescence detection is detected if a fluorescence intensity is below the predetermined threshold.

Embodiment 3 is a computer-readable medium comprising: instructions that when executed by a processor of a nucleic acid amplification system cause the nucleic acid amplification system to perform a method of monitoring fluorescence signals from a nucleic acid amplification reaction for abnormal fluorescence detection, the method comprising:
a) measuring fluorescence signal from the nucleic acid amplification reaction at a plurality of time points in a time period during which the nucleic acid amplification reaction is occurring;
b) determining a plurality of fluorescence intensities at at least a portion of the plurality of time points from the measured fluorescence signals;
c) comparing each of the plurality of fluorescence intensities to a predetermined threshold, wherein abnormal fluorescence detection is detected if a fluorescence intensity is below the predetermined threshold.

Embodiment 4 is the method, system, or computer-readable medium of any one of the preceding embodiments, wherein the nucleic acid amplification reaction is a thermocycled nucleic acid amplification reaction.

Embodiment 5 is the method, system, or computer-readable medium of the immediately preceding embodiment, wherein the time period begins after commencement of thermocycling, or after the first thermocycle.

Embodiment 6 is the method, system, or computer-readable medium of any one of the preceding embodiments, wherein the plurality of fluorescence intensities are determined by smoothing the fluorescence signals measured at the plurality of time points.

Embodiment 7 is the method, system, or computer-readable medium of any one of the preceding embodiments, wherein the plurality of fluorescence intensities are determined as a moving average or moving median of the fluorescence signals measured at the plurality of time points.

Embodiment 8 is the method, system, or computer-readable medium of any one of the preceding embodiments, wherein the fluorescence signal is measured by a fluorescence detector, optionally wherein the fluorescence detector comprises a photomultiplier or photodiode.

Embodiment 9 is the method, system, or computer-readable medium of the immediately preceding embodiment, wherein a defect in the fluorescence detector is detected if a fluorescence intensity is below the predetermined threshold.

Embodiment 10 is the method, system, or computer-readable medium of any one of the preceding embodiments, wherein the nucleic acid amplification reaction is contained within a microfluidic cartridge.

Embodiment 11 is the method, system, or computer-readable medium of the immediately preceding embodiment, wherein the microfluidic cartridge comprises:
a) a plurality of functional areas comprising a sample preparation area, a nucleic acid amplification area, and a waste area;
b) a central distribution hub; and
c) a pump, a plurality of valves, and a fluidic network of microchannels connecting the functional areas to the hub;
wherein the pump, plurality of valves, and fluidic network of microchannels can drive movement of a fluid from a first functional area through the central distribution hub to a second functional area of the plurality of functional areas; and the nucleic acid amplification reaction is within the nucleic acid amplification area.

Embodiment 12 is the method, system, or computer-readable medium of any one of the preceding embodiments, wherein fluorescence signals are measured from a plurality of nucleic acid amplification reactions in step a).

Embodiment 13 is the method, system, or computer-readable medium of the immediately preceding embodiment, wherein step b) comprises determining a plurality of fluorescence intensities at at least a portion of the plurality of time points from the measured fluorescence signals for each of the plurality of nucleic acid amplification reactions.

Embodiment 14 is the method, system, or computer-readable medium of the immediately preceding embodiment, wherein step c) comprises comparing each of the plurality of fluorescence intensities to a predetermined threshold for each of the pluralities of fluorescence intensities corresponding to the plurality of nucleic acid amplification reactions.

Embodiment 15 is the method, system, or computer-readable medium of any one of embodiments 12-14, wherein the plurality of fluorophore-containing nucleic acid amplification reactions is contained in a multi-well plate or in a plurality of tubes.

Embodiment 16 is the method, system, or computer-readable medium of any one of the preceding embodiments, wherein the nucleic acid amplification reaction comprises a fluorophore.

Embodiment 17 is the method, system, or computer-readable medium of the immediately preceding embodiment, wherein the fluorophore is associated with an oligonucleotide probe, optionally wherein the oligonucleotide probe further comprises a quencher.

Embodiment 18 is the method, system, or computer-readable medium of any one of embodiments 1-15, wherein the nucleic acid amplification reaction does not comprise a fluorophore associated with an oligonucleotide and fluorescence signal comprising inherent fluorescence from one or more reagents or templates in the nucleic acid amplification reaction is measured, optionally wherein the one or more reagents comprise dNTPs and/or one or more primers.

Embodiment 19 is the method of any one of embodiments 1 or 4-18, wherein a fluorescence intensity is below the predetermined threshold and abnormal fluorescence detection is detected.

Embodiment 20 is the method of embodiment 19, wherein the method further comprises discontinuing the nucleic acid amplification reaction after detecting the abnormal fluorescence detection.

Embodiment 21 is the method of any one of embodiments 1 or 4-18, wherein each of the plurality of fluorescence intensities are above the predetermined threshold and no abnormal fluorescence detection is detected.

Embodiment 22 is the system of any one of embodiments 2 or 4-17, wherein the system is configured to discontinue the nucleic acid amplification reaction if abnormal fluorescence detection is detected.

Embodiment 23 is the system or computer-readable medium of any one of embodiments 2-17 or 22, wherein the method further comprises discontinuing a nucleic acid amplification reaction if an abnormality is detected.

Additional objects and advantages will be set forth in part in the description which follows, and in part will be understood from the description, or may be learned by . practice. The objects and advantages will be realized and attained by means of the elements and combinations particularly pointed out in the appended claims.

It is to be understood that both the foregoing general description and the following detailed description are exemplary and explanatory only and are not restrictive of the claims.

### BRIEF DESCRIPTION OF THE DRAWINGS

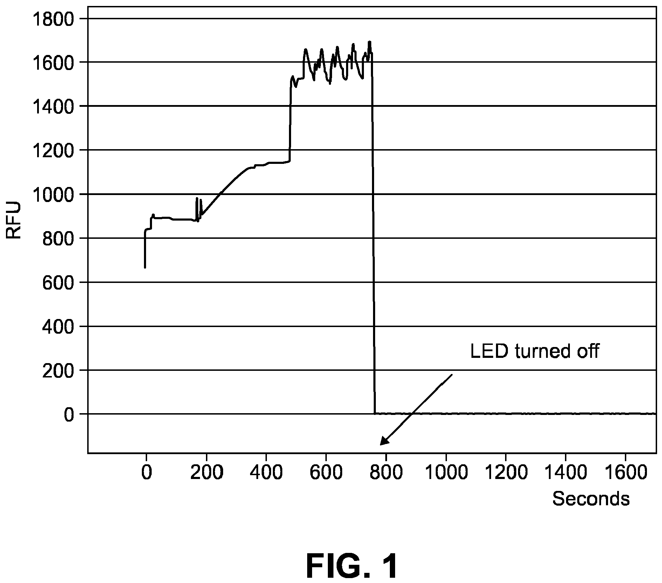

Figure 1 is an exemplary graph of fluorescence over time from a nucleic acid amplification reaction. Normal conditions of qPCR provide a non-null fluorescence intensity (measured in RFU, relative fluorescence units). The light source responsible for fluorescence excitation, an LED, was turned off at the indicated time to simulate an abnormality such as failure of the light source or the detector. The measured fluorescence intensity dropped substantially below the fluorescence intensities measured before the LED was turned off. Relative fluorescence units (signal; RFUs; y-axis) are plotted versus time (seconds on the x-axis):

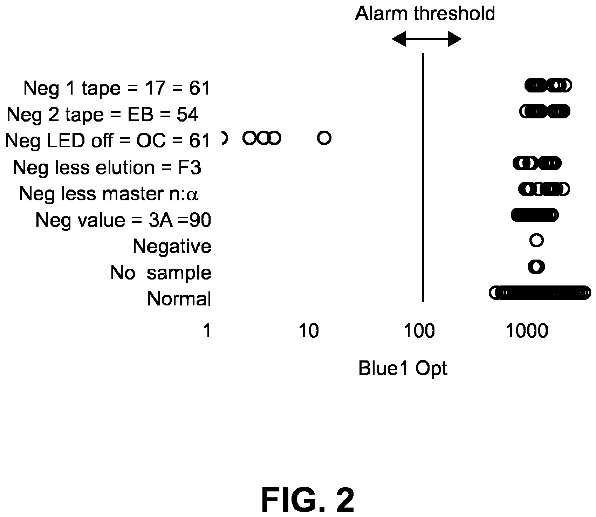

Figure 2 shows exemplary distributions of minimum fluorescence intensities (determined as 5-point averages of individual measurements) for 2 qPCR channels from a plurality of reactions under normal and various abnormal conditions. Each circle represents a fluorescence measurement (measured in RFUs) observed in a single nucleic amplification run. The "NEG LED off' condition shows the results when the LED light source was inoperative during the run, representing abnormal fluorescence detection. A threshold can be set for each channel as illustrated, e.g., at approximately 100 RFUs (shown as a vertical line), to distinguish abnormal fluorescence detection from normal conditions and other abnormalities unrelated to fluorescence detection.

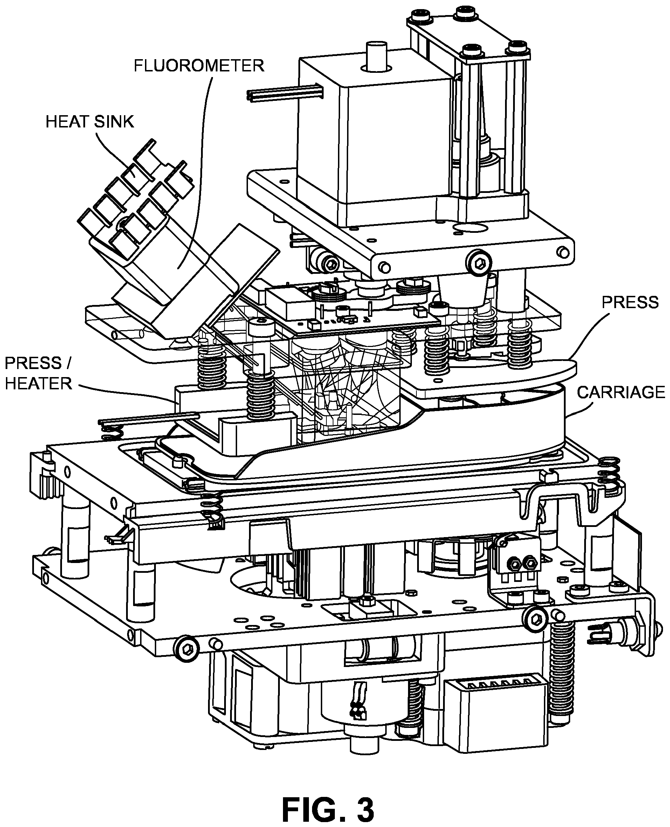

Figure 3 is an exemplary system capable of nucleic acid amplification loaded with a microfluidic cartridge in its docking station. The system includes a heat sink, a fluorometer, and a press/ heater used for interacting and measuring a sample loaded in the cartridge.

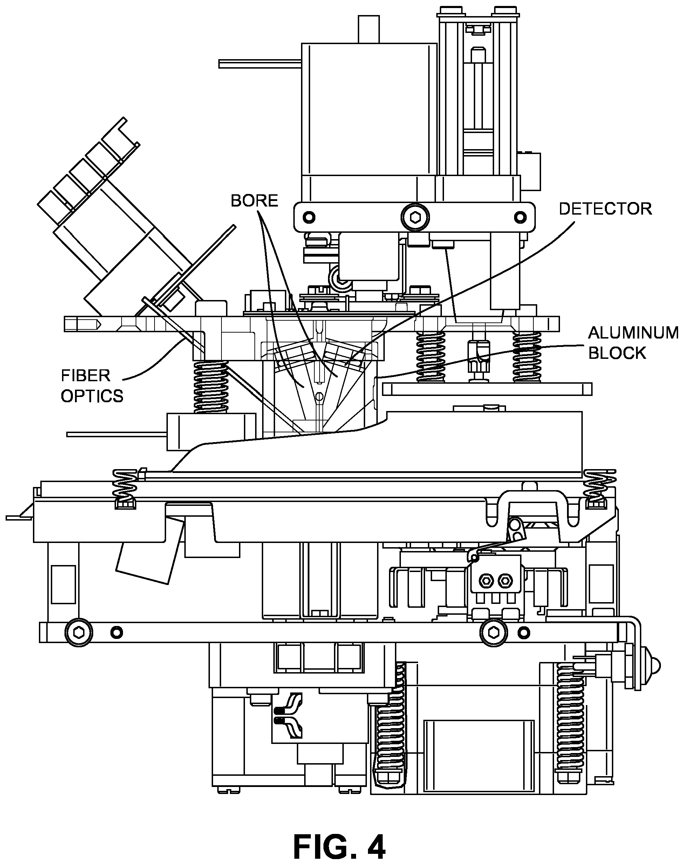

Figure 4 is a side view of the docking station of Figure 3. The side view shows the fiber optics that run from the cartridge to the fluorometer as well as the detector, bore and aluminum block used for taking fluorescence measurements.

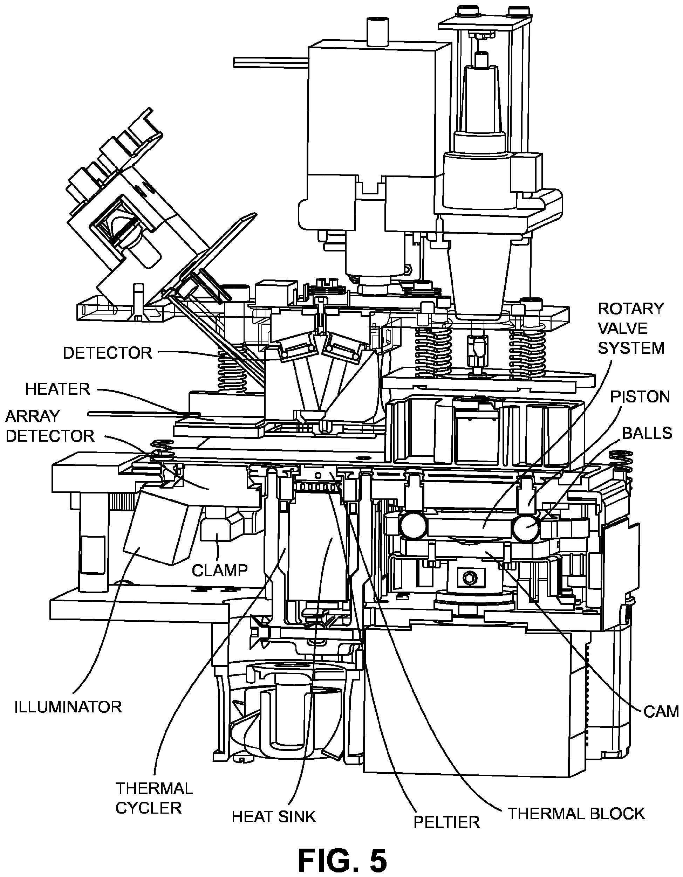

Figure 5 shows a cutaway view of the nucleic acid amplification system docking station and a microfluidic cartridge of Figures 3 and 4. The cutaway view shows internal components such as the thermal cycler, thermal block, array detector, rotary valve system and pistons useful for moving and analyzing liquids in different functional areas of the cartridge.

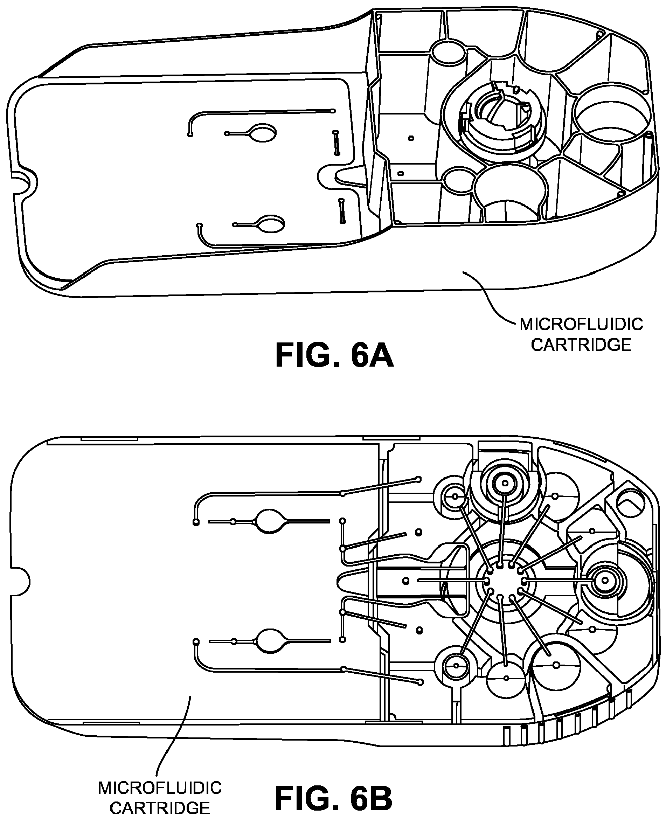

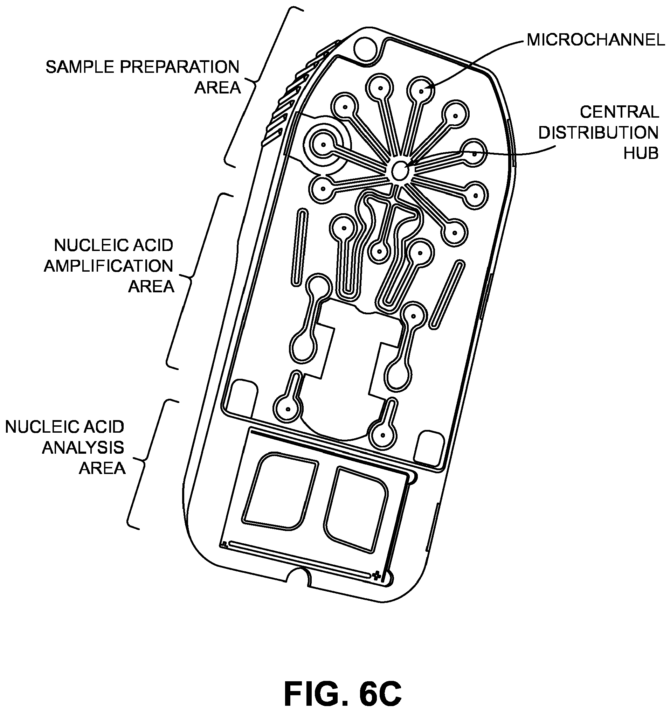

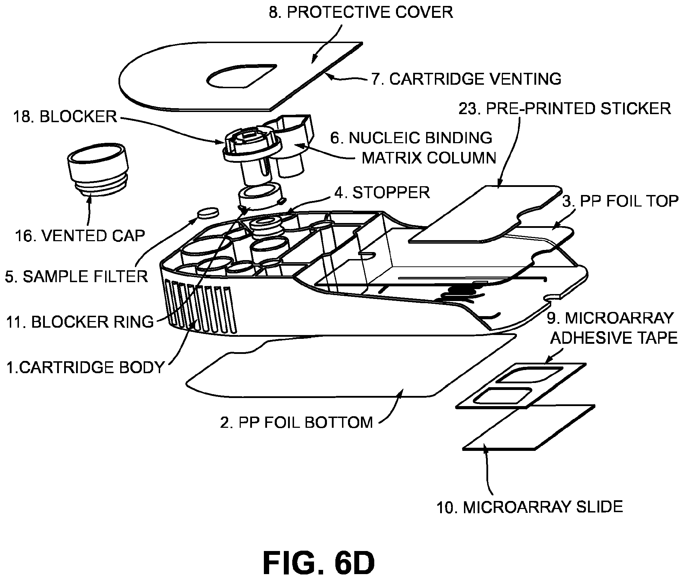

Figures 6A-6D show various views of an exemplary microfluidic cartridge. Figure 6A shows a top-down view of a microfluidic cartridge which contains multiple chambers for housing samples, reagents, or other liquids. Figure 6B shows the bottom view of the exemplary microfluidic cartridge which shows a fluidic network of microchannels connecting various chambers to other areas within the cartridge. Figure 6C shows a bottom view of a fully constructed microfluidic cartridge which includes a sample preparation area, a nucleic acid amplification area, and a nucleic acid analysis area. These functional areas are connected by the series of microchannels shown in detail in Figure 6B. The sample preparation area includes liquids in adjacent chambers. The system can be programmed to combine liquids contained in these chambers in discrete volumes in a particular order. When docked in the system, the nucleic acid amplification area is in close proximity to the thermal cycler in Figure 4. Similarly, when docked in the system, the nucleic acid analysis area is in close proximity to the array detector as shown in Figure 5. Figure 6D shows an exploded view of the fully constructed exemplary microfluidic cartridge which includes a cartridge body (1) a vented cap for covering the sample after input (16), a sample filter (5) and the components for the cartridge to interact with the mechanical components of the system to move and combine liquids in the sample preparations area (4, 6-8, 11). The exploded view also shows how the microarray slide (10) is attached to the nucleic acid amplification area via an adhesive tape (9) in the nucleic amplification analysis area. The cartridge also includes polypropylene (PP) foil coverings (2, 3).

### DEFINITIONS

Unless defined otherwise, all technical and scientific terms used herein have the same meaning as commonly understood by one of ordinary skill in the art pertinent to the methods and compositions described. All patents, applications, published applications and other publications referred to herein are incorporated by reference in their entirety. If a definition set forth in this section is contrary to or otherwise inconsistent with a definition set forth in the patents, applications, published applications, and other publications that are herein incorporated by reference, the definition set forth in this section prevails over the definition that is incorporated herein by reference.

A "fluorescence detector" or "fluorescence sensor" as used herein, refers to an optical detector that is capable of obtaining fluorescence measurements. Such measurements can be obtained prior to, during and after nucleic acid amplification. A fluorescence detector may be a photodiode or photomultiplier. The fluorescence detector receives light (fluorescence) emitted from a sample.

"Nucleic acid" and "polynucleotide" refers to a multimeric compound including two or more covalently bonded nucleosides or nucleoside analogs having nitrogenous heterocyclic bases, or base analogs, where the nucleosides are linked together by phosphodiester bonds or other linkages to form a polynucleotide. Nucleic acids include RNA, DNA, and combinations and analogs thereof such as "peptide nucleic acids" or PNAs (see, e.g., WO 95/32305) and "locked nucleic acids" (LNA), in which one or more nucleotide monomers have a bicyclic furanose unit locked in an RNA mimicking sugar conformation (see, e.g., Vester et al., Biochemistry 43:13233-41, 2004). Nitrogenous bases may be conventional bases (A, G, C, T, U), analogs thereof (e.g., inosine, 5-methylisocytosine, isoguanine; see, e.g., The Biochemistry of the Nucleic Acids 5-36, Adams et al., ed., 11th ed., 1992; Abraham et al., 2007, BioTechniques 43: 617-24), which include derivatives of purine or pyrimidine bases (e.g., N⁴-methyl 8deoxyguanosine, deaza- or aza-purines, deaza- or aza-pyrimidines, etc.; U.S. Pat. Nos. 5,378,825, 6,949,367 and International Patent Application Pub. No. WO 93/13121), and/or "abasic" residues (see. e.g., U.S. Pat. No. 5,585,481).

A "target" material as used herein is a material to be detected or quantified. A target material may be a nucleic acid; other embodiments of target materials include cells, viruses, and other biomolecules. A "target nucleic acid" as used herein is a nucleic acid including a target sequence to be detected or quantified, e.g., via amplification. Target nucleic acids or target nucleic acids of interest may be DNA or RNA or combinations or analogs thereof as described herein and may be either single-stranded or double-stranded. The target nucleic acid may include other sequences besides the target sequence, which may not be detected or quantified.

The term "region," as used herein, refers to a portion of a nucleic acid wherein said portion may be less than the entire nucleic acid. For example, the term "region" may be used to refer to a smaller target-hybridizing portion of the entire oligonucleotide. Certain oligonucleotides, such as primers, may consist entirely of one region (e.g., target-hybridizing region) or may contain a plurality of regions (e.g., a promoter sequence region and a target-hybridizing region).

"Sample" refers to any composition that may contain or is suspected of containing a target material. A sample may be a complex mixture of components. Samples include "biological samples" which include any tissue or material derived from a living or dead mammal or organism, including, for example, stool, blood, plasma, serum, blood cells, saliva, mucous and cerebrospinal fluid. Samples may also include samples of in vitro cell culture constituents including, for example, conditioned media resulting from the growth of cells and tissues in culture medium. Samples also include food, which includes any material intended or suitable for consumption, including solids, suspensions, emulsions, gels, and liquids (thus including gelatin, milk, soups, beverages, ice-cream, fruit smoothies, emulsified cheese dips, fruit puree, nut butter, processed and/or textured protein, and the like, as well as bread, fruit, vegetables, and meat). Samples also include water and aqueous solutions. A sample may be treated chemically, physically, or mechanically to disrupt tissue or cell structure to release intracellular nucleic acids into a solution. Samples may be treated to release nucleic acids into a solution containing enzymes, buffers, salts, detergents and the like.

The interchangeable terms "oligomer," "oligo," and "oligonucleotide" refer to a nucleic acid having generally less than 1,000 nucleotide (nt) residues, including polymers in a range having a lower limit of about 5 nt residues and an upper limit of about 500 to 900 nt residues. In some embodiments, oligonucleotides are in a size range having a lower limit of about 12 to 15 nt and an upper limit of about 50 to 600 nt, and other embodiments are in a range having a lower limit of about 15 to 20 nt and an upper limit of about 22 to 100 nt. An oligonucleotide may serve one or more of various different functions, e.g., as a primer and/or promoter, detection probe, capture oligomer, etc.

"Primer," "amplification oligonucleotide" or "oligonucleotide primer" refers to a polynucleotide, generally an oligonucleotide including a "target" binding portion, that is designed to selectively hybridize with a target nucleic acid flanking sequence or to a corresponding primer-binding site of an amplification product under appropriate stringency conditions and serve as the initiation point for the synthesis of a nucleotide sequence that is complementary to the corresponding polynucleotide template from its 3'-end.

"Amplifying" or "amplification" refers to any known procedure for obtaining multiple copies of a target nucleic acid sequence or its complement or fragments thereof. The multiple copies may be referred to as amplicons or amplification products. Known amplification methods include both thermal cycling and isothermal amplification methods. Polymerase chain reaction (PCR), replicase-mediated amplification, ligase chain reaction (LCR), strand-displacement amplification (SDA), and transcription-associated amplification (e.g., transcription-mediated amplification (TMA) or NASBA) are non-limiting examples of nucleic acid amplification methods. See, e.g., US Pat. Nos. 4,868,105; 5,124,246; 5,130,238; 5,399,491; 5,437,990; 5,554,516; and 7,374,885; and PCT Pub. Nos. WO 88/01302; WO 88/10315 and WO 95/03430 (TMA); US Pat. No. 4,786,600 (RCA); US Pat. No. 5,427,930 and US Pat. No. 5,516,663 (LCR); and US Pat. No. 5,422,252; US Pat. No. 5,547,861; and US 5,648,211 (SDA). See also, e.g., Compton, Nature 350:91-92, 1991; Malek et al., Methods Mol. Biol. 28:253-260, 1994 (NASBA). Briefly, PCR amplification uses a DNA polymerase, pairs of primers, and thermal cycling to synthesize multiple copies of two complementary strands from dsDNA or from a cDNA (see, e.g., US Pat. Nos. 4,683,195, 4,683,202, and 4,800,159).

As used herein, the term "real-time amplification" refers to amplification of target nucleic acid that is monitored by real-time detection. Real-time PCR amplification includes, e.g., what is commonly referred to as Taqman^{®} PCR (see, e.g., Holland et al., Proc. Natl. Acad. Sci. USA 88:7276-7280, 1991; and Livak et al., US Pat. No. 6,030,787). Taqman PCR is a type of real-time PCR that uses a nucleic acid probe complementary to an internal segment of the target DNA. The probe is labeled with two fluorescent moieties. The emission spectrum of one overlaps the excitation spectrum of the other, resulting in "quenching" of the first fluorophore by the second.

As used herein, "thermocycling" is a process of cyclically heating and cooling of a nucleic acid amplification mixture that facilitates amplifying nucleic acids through successive rounds of denaturation, primer annealing, and primer extension, e.g., by a thermostable polymerase. In many examples, thermocycling includes holding a reaction mixture at two or more different temperatures, each for a predetermined length of time, and performing several cycles of those two or more temperatures to cause nucleic acid amplification. A nucleic, acid amplification reaction mixture that has been subjected to thermocycling is termed a "thermocycled reaction mixture."

As used herein, a "nucleic acid amplification system" refers to a device or apparatus that may be used to perform nucleic acid amplification. In many examples, the nucleic acid amplification system includes a fluorescence detector and is capable of obtaining fluorescence measurements prior to, during and after nucleic acid amplification. In many examples, the nucleic acid amplification system includes a temperature controller and is capable of providing or transferring heat to nucleic acid amplification reaction mixtures via one or more heating elements, which may include, e.g., a heat block. The nucleic acid amplification system is typically programmable and can hold temperatures for different lengths of time. The nucleic acid amplification system may be configured to accommodate any one or more of a variety of reaction vessels, such as tubes, multi-well strips, multi-well plates, microfluidic chips, and microfluidic cartridges, which may contain one or more nucleic acid amplification mixtures.

The term "amplicon" or the term "amplification product" as used herein refers to the nucleic acid molecule generated during an amplification procedure that is complementary or homologous to a sequence contained within the target sequence. These terms can be used to refer to a single-stranded amplification product, a double-stranded amplification product, or one of the strands of a double-stranded amplification product.

By "complementary" is meant that the nucleotide sequences of similar regions of two single-stranded nucleic acids, or two different regions of the same single-stranded nucleic acid, have nucleotide base compositions that allow the single-stranded regions to hybridize together in a stable double-stranded hydrogen-bonded region under stringent hybridization or amplification conditions. Sequences that hybridize to each other may be completely complementary or partially complementary to the intended target sequence by standard nucleic acid base pairing (e.g., G:C, A:T, or A:U pairing). By "sufficiently complementary" is meant a contiguous sequence that is capable of hybridizing to another sequence by hydrogen bonding between a series of complementary bases, which may be complementary at each position in the sequence by standard base pairing or may contain one or more residues, including abasic residues, that are not complementary. Sufficiently complementary contiguous sequences typically are at least 80%, or at least 90%, complementary to a sequence to which an oligomer is intended to specifically hybridize. Sequences that are "sufficiently complementary" allow stable hybridization of a nucleic acid oligomer with its target sequence under appropriate hybridization conditions, even if the sequences are not completely complementary. When a contiguous sequence of nucleotides of one single-stranded region is able to form a series of "canonical" or "Watson-Crick" hydrogen-bonded base pairs with an analogous sequence of nucleotides of the other single-stranded region, such that A is paired with U or T and C is paired with G, the nucleotides sequences are "completely" complementary (see. E.g., Sambrook et al., Molecular Cloning: A Laboratory Manual, 2nd ed. (Cold Spring Harbor Laboratory Press, Cold Spring Harbor, N. Y., 1989) at §§1.90-1.91, 7.37-7.57, 9.47-9.51 and 11.47-11.57, particularly §§9.50- 9.51, 11.12-11.13, 11.45-11.47 and 11.55-11.57). Appropriate hybridization conditions are well-known in the art, may be predicted based on sequence composition, or can be determined by using routine testing methods (see e.g., Sambrook et al., supra.)

As used herein, a "label" or "detection label" refers to a moiety or compound that can be detected or generate a detectable signal and that is joined directly or indirectly to a molecule, such as a probe. Direct labeling can occur through bonds or interactions that link the label to the molecule, including covalent bonds or non-covalent interactions, e.g., hydrogen bonds, hydrophobic and ionic interactions, or formation of chelates or coordination complexes. Indirect labeling can occur through use of a bridging moiety or "linker" such as a binding pair member, an antibody or additional oligomer, which is either directly or indirectly labeled, and which may amplify the detectable signal. Labels include any detectable moiety, such as a radionuclide, ligand (e.g., biotin, avidin), enzyme or enzyme substrate, reactive group, or chromophore (e.g., dye, particle, or bead that imparts detectable color), luminescent compound (e.g., bioluminescent, phosphorescent, or chemiluminescent labels), or fluorophore. Common labels used for TaqMan^{®} probes include a fluorophore and a quencher. "Fluorophore" as used herein refers to any label whose presence can be detected by its fluorescent light emitting properties. The term "quencher" as used herein refers to a moiety that absorbs at least some of the intensity of a fluorescent emission. Quenchers include fluorescent quenchers and dark quenchers (sometimes also referred to as non-fluorescent quenchers). A dark quencher is a substance that absorbs excitation energy from a fluorophore and dissipates the energy as heat; while a fluorescent quencher re-emits much of this energy as light. A fluorescent quencher is a moiety, typically a fluorophore, that can absorb the fluorescent signal emitted from a source of fluorescence at a first wavelength, for example but not limited to, a nucleic acid dye associated with a double-stranded segment of nucleic acid, and after absorbing enough fluorescent energy, the fluorescent quencher can emit fluorescence at a second wavelength that is characteristic of the quencher, a process termed "fluorescent resonance energy transfer" or FRET. Exemplary fluorophores include FAM, SYBR^{®} Green, ATTO fluorescent labels, VIC, JOE, NED, Cy3, ROX, Texas Red and Cy5 dyes (all available from numerous commercial sources). Exemplary quenchers include BBQ, ATTO quenchers, BHQ, TAMRA and DABCYL (all available from numerous commercial sources). Synthesis and methods of attaching labels to nucleic acids and detecting labels are known in the art (see for example, Sambrook et al., Molecular Cloning, A Laboratory Manual, 2nd ed. (Cold Spring Harbor Laboratory Press, Cold Spring Harbor, NY, 1989), Chapter 10; US Pat. Nos. 5,658,737, 5,656,207, 5,547,842, 5,283,174, and 4,581,333). More than one label, and more than one type of label, may be present on a particular probe, or detection may use a mixture of probes in which each probe is labelled with a compound that produces a different detectable signal (see, e.g., US Pat. Nos. 6,180,340 and 6,350,579).

"Detection probe," "detection oligonucleotide," "detection oligomer," "probe oligomer," and "detection probe oligomer" are used interchangeably to refer to a nucleic acid oligomer that hybridizes specifically to a target sequence in a nucleic acid, such as an amplified nucleic acid, under conditions that promote hybridization to allow detection of the target sequence or amplified nucleic acid. Detection may either be direct (e.g., a probe hybridized directly to its target sequence) or indirect (e.g., a probe linked to its target via an intermediate molecular structure). Detection probes may be DNA, RNA, analogs thereof, or combinations thereof (e.g., DNA/RNA chimerics) and they may be labeled or unlabeled. Detection probes may further include alternative backbone linkages such as, e.g., 2'-O-methyl linkages. A detection probe's "target sequence" generally refers to a smaller nucleic acid sequence region within a larger nucleic acid sequence that hybridizes specifically to at least a portion of a probe oligomer by standard base pairing. A detection probe may include target-specific sequences and other sequences that contribute to the three-dimensional conformation of the probe (see, e.g., U.S. Pat. Nos. 5,118,801; 5,312,728; 6,849,412; 6,835,542; 6,534,274; and 6,361,945; and US Patent Application Pub. No. 20060068417).

An "elution buffer" as used herein is a suitable liquid for separating nucleic acids from a solid support.

A "master mix buffer," "master mix," or "amplification master mix buffer" as used herein includes amplification reagents and optionally primers used to amplify a target nucleic acid but does not include the sample to be amplified.

An "amplification reaction chamber," also referred to as "reaction chamber" or "reaction space" denotes a space in which the amplification of target nucleic acids, is performed. Multiple amplification reaction chambers may be arranged in parallel or substantially parallel to each other in a variety of vessels. Examples of vessels that can contain multiple amplification reaction chambers include a multi-well strip, a multi-well plate, a microfluidic chip, or a microfluidic cartridge.

As used herein, the term "relative fluorescence unit" ("RFU") is a unit of measurement of fluorescence intensity. RFU varies with the characteristics of the detection equipment used for the measurement and can be used as a measurement to compare relative intensities between samples and controls.

"Smoothing" as used herein in the context of data analysis, refers to a technique for reducing noise in data by adjusting a measured value to reduce or eliminate noise in the measurement, generally to be closer to surrounding measurements. Smoothing creates an approximating function that attempts to capture important patterns in the data, while leaving out noise. Examples of smoothing include moving average/median, local regression (loess), low pass filter, a Savitzky-Golay smoothing filter, and or a Ramer-Douglas-Peucker algorithm.

As used herein, the term "substantially" can be synonymous with the term "essentially" and indicates that a step, reagent, component, or other element achieves a result or has a property that does not materially differ, but may have minor differences or deviations, from the result or property to which "substantially" applies. For example, to "substantially avoid variation" may mean that the variation is less than or equal to about 20%, 15%, 10%, 5%, 4%, 3%, 2%, or 1%.

As used herein, the term "about" refers to a numerical value, including, for example, whole numbers, fractions, and percentages, whether or not explicitly indicated. When the term precedes a list of numerical values or ranges, the terms modify all of the values or ranges. As applied to measured quantities, the term includes the exact numerical value modified by the term, as well as ranges of values that would be expected to be within experimental error. For example, "about 5°C" means "5°C" and a range of temperatures that is within experimental error, e.g., plus or minus (±) 20% of 5°C, ±15% of 5°C, ±10% of 5°C, or ±5% of 5°C. The term has a similar meaning with respect to other parameters. For example, "about 5 minutes" means "5 minutes" and a range of times that is within experimental error, e.g., plus or minus (±) 20% of 5 minutes, ±15% of 5 minutes, ±10% of 5 minutes, or ±5% of 5 minutes. In some contexts, the percentage of experimental error is implied or apparent and may not be explicitly stated. In some contexts, the percentage of experimental error will be provided explicitly. The term "about" may be used to modify any measurable quantity, including quantities of time, temperature, volume, mass, weight, length, density, size, percentage, ratio, dose, frequency, pressure, rate, and intensity. In some instances, the term about may include numerical values that are rounded to the nearest significant figure.

Reference to a numerical range herein (e.g., "X to Y" or "from X to Y" or "between X and Y") includes the endpoints defining the range and all values falling within the range.

The terms "a," "an," and "the" include plural referents, unless the context clearly indicates otherwise. For example, "a nucleic acid" as used herein is understood to represent one or more nucleic acids. As such, the terms "a" (or "an"), "one or more," and "at least one" can be used interchangeably herein.

"Or" is used in the inclusive sense, i.e., is equivalent to "and/or," unless the context clearly indicates otherwise.

### DETAILED DESCRIPTION

Disclosed herein are methods for fluorescence signals from a nucleic acid amplification reaction for abnormal fluorescence detection. The methods can leverage the presence of fluorophores, quenchers (substances capable of affecting fluorescent emissions by static or dynamic quenching), and/or reagents with inherent fluorescence, e.g., dNTPs and primers, to detect abnormal fluorescence detection while monitoring fluorescence signals from a nucleic acid amplification reaction. Typically, an abnormal fluorescence detection correlates with failure of a light source and/or fluorescence detector. The abnormalities may adversely affect the reliability of the data collected from the reaction. Importantly, the methods can be implemented on many existing nucleic acid amplification systems, such as real-time nucleic acid amplification systems and any other systems equipped with a fluorescence detector, without requiring additional hardware. Instead, the methods use the existing features of such systems to monitor fluorescence signals for abnormal detection during a nucleic acid amplification reaction.

### A. Detection Labels and Probes

Detection labels (e.g., fluorophores) may be used in accordance with the present disclosure. Nucleic acid amplification reaction mixtures used in methods of the present disclosure may include detection oligomers (e.g., designed to hybridize to an amplicon) and/or amplification oligomers, such as forward and/or reverse primers, with detection labels. In general, an amplification oligomer or detection oligomer with a detection label used in an amplification reaction includes at least (1) a region for hybridizing specifically to a region on a target nucleic acid sequence and (2) a detection label. In some embodiments, an oligomer with a detection label is termed a detection probe.

Suitable fluorophores for use as detection labels can include compounds that emit a detectable light signal, e.g., fluorophores ("fluorescent dye compounds"). More than one label, and more than one type of label, may be present on a particular probe, or a mixture of probes may be used in which each probe is labeled with a compound that produces a detectable signal (see, e.g., US Pat. Nos. 6,180,340 and 6,350,579). Labels may be attached to a probe by various means including covalent linkages, chelation, and ionic interactions, but preferably the label is covalently attached. Suitable fluorophores are well-known in the art and include, for example, CAL Flour^{®} Orange 560, CAL Flour^{®} Red 610, FAM, or ATTO 490LS. In some embodiments, the fluorophore is a temperature-sensitive fluorophore. In some embodiments, the temperature-sensitive fluorophore is sulforhodamine. In embodiments including fluorophore-labeled detection probes, each detection probe further includes a quencher. Suitable quenchers are well-known in the art and include, for example, BHQ, TAMRA, and DABCLY. In other embodiments, a detection probe includes both a fluorescent label and a quencher, a combination that is particularly useful in fluorescence resonance energy transfer (FRET) assays. Specific variations of such detection probes include, e.g., a TaqMan^{®} detection probe (Roche Molecular Diagnostics), a "molecular beacon" (see, e.g., Tyagi et al., Nature Biotechnol. 16:49-53, 1998; US Patent Nos. 5,118,801 and 5,312,728), and a "molecular torch" (see, e.g., US Patent Nos. 6,849,412; 6,835,542; 6,534,274; and 6,361,945). In some embodiments, the fluorophore in a nucleic acid amplification reaction mixture is associated with a probe that further includes a quencher, such as a TaqMan^{®} probe.

### B. Reaction Mixtures

In some embodiments, a reaction mixture used in methods or systems described herein includes one or more of the amplification oligomers for amplification of a target nucleic acid. In some embodiments, the amplification oligomers include detection labels. The reaction mixture will typically include other reagents suitable for performing *in vitro* amplification such as buffers, salt solutions, appropriate nucleotide triphosphates (e.g., dATP, dCTP, dGTP, dTTP, ATP, CTP, GTP and UTP), and/or enzyme(s) (e.g., DNA polymerase, reverse transcriptase, and RNA polymerase), and may include test sample components, in which an internal control (IC) target nucleic acid may be present.

### C. Reaction Chambers; Samples

A variety of reaction vessels may be used to contain the nucleic acid amplification reactions used according to the present disclosure. A reaction vessel may contain a reaction chamber or a plurality of reaction chambers. In some embodiments, the steps of the methods disclosed herein are performed with nucleic acid reaction chambers. In some embodiments, the steps of the methods disclosed herein are performed with nucleic acid reaction chambers that are empty, or with nucleic acid reaction chambers containing nucleic acid amplification reaction mixtures. In some embodiments, a plurality of fluorophore-containing nucleic acid amplification reaction mixtures is contained in a plurality of wells or a plurality of tubes.

Nucleic acid reaction chambers may be configured into a variety of reaction vessels with each reaction vessel including a plurality of reaction chambers. In some embodiments, a reaction vessel includes a plurality of reaction chambers that are empty and/or reaction chambers that contain nucleic acid amplification reaction mixtures. In some embodiments, the vessel is a multi-chambered receptacle. In some embodiments, the nucleic acid amplification chamber is contained within a multi-chambered receptacle. Non-limiting examples of vessels include multi-well strips, multi-well plates, microfluidic chips, and microfluidic cartridges. In some embodiments, a plurality of nucleic acid amplification reaction mixtures is contained in a multi-well plate or in a plurality of tubes.

In some embodiments, at least one nucleic acid amplification reaction is contained within in a multi-well strip, a multi-well plate, a microfluidic chip, or a microfluidic cartridge. In some embodiments, a plurality of fluorophore-containing nucleic acid amplification reactions is contained in a multi-well plate or in a plurality of tubes. In some embodiments, the reaction vessel is a microfluidic cartridge. In some embodiments the microfluidic cartridge, also referred to as a "lab-on-a-chip," can perform a complete nucleic acid analysis of a sample, from sample collection to nucleic acid amplification, to the reading of the result(s). Exemplary microfluidic cartridges that can be used to perform the steps of the methods disclosed herein are depicted in Figures 6A-6D. Figure 6A shows a top-down view of an exemplary microfluidic cartridge which contains multiple chambers for housing samples, reagents, or other liquids. Microchannels connect such chambers to move liquids among different functional areas within the cartridge. Figure 6B shows the bottom view of the exemplary microfluidic cartridge which shows a network of microchannels connecting various chambers to other areas within the cartridge. Figure 6C shows a bottom view of a fully constructed microfluidic cartridge which includes a sample preparation area, a nucleic acid amplification area, and a nucleic acid analysis area. These functional areas are connected by the series of microchannels shown in detail in Figure 6B. The sample preparation area includes liquids in adjacent chambers. A system, e.g., a nucleic acid amplification system, can be programmed to combine liquids contained in these chambers in discrete volumes in a particular order. When docked in the system, the nucleic acid amplification area is in close proximity to a thermal cycler, as shown in Figure 4. Similarly, when docked in a system, a nucleic acid analysis area is in close proximity to an array detector as shown in Figure 5. Figure 6D shows an exploded view of the fully constructed exemplary microfluidic cartridge which includes a cartridge body (1) a vented cap for covering the sample after input (16), a sample filter (5) and the components for the cartridge to interact with the mechanical components of the system to move and combine liquids in the sample preparations area (4, 6-8, 11). The exploded view of Figure 6D also shows how the microarray slide (10) is attached to the nucleic acid amplification area via an adhesive tape (9) in the nucleic amplification analysis area. The cartridge also includes polypropylene (PP) foil coverings (2, 3).Microfluidic cartridges have several advantages, including the capability to conduct automated operations while consuming low reagent volumes, while being inexpensive and disposable. Examples of microfluidic cartridges are also disclosed in US Patent No. 10,654,039.

In some embodiments, a microfluidic cartridge includes at least a) a plurality of functional areas including a sample preparation area, a nucleic acid amplification area, a nucleic acid analysis area, and a waste area; b) a central distribution hub; and c) a pump, a plurality of valves, and a fluidic network of microchannels connecting the functional areas to the central distribution hub. The pump, plurality of valves, and fluidic network of microchannels can drive movement of a fluid from a first functional area through the central distribution hub to a second functional area of the plurality of functional areas. The nucleic acid amplification reaction is contained within the nucleic acid amplification area.

The sample preparation area may include of a network of reservoirs or tanks holding a plurality of liquids or gases in various configurations, as shown in Figure 6A. Such liquids or gases may contain a sample and/or reagent commonly used in the art, e.g., master mix, wash buffer, elution buffers hybridization buffers. Arrangement, distribution, and transfer of such reagents may be customized based on experimental protocols or amplification systems used.

The nucleic acid amplification area may be adjacent to the sample preparation area and connected via microfluidic channels. Such channels allow for the movement of prepared samples to the nucleic acid amplification area through the central distribution hub to be subjected to nucleic acid amplification conditions.

The nucleic acid analysis area may include a microarray component or slide, as shown in Figure 6C. A microarray slide may be used to analyze the amplified nucleic acids via capture probes. Such microarrays may include an array of many individual fragments of DNA immobilized on a solid support (e.g., glass slide) that hybridize with complementary target sequences in an organism of interest. Hybridization may be detected using a fluorescent reporter molecule, e.g., a fluorophore. The inclusion of different probe sequences on one microarray allows simultaneous detection of different organisms, or differences between organisms of the same species, allowing for syndromic testing with a high degree of specificity.

A functional area may be a space dedicated to a specific operation on the sample. The functional areas of a microfluidic cartridge may be fluidly connected to a central distribution hub by a fluidic network of microchannels. Functional areas can be arranged in a variety of ways, and multiple functional areas can be either identical to or different from each other. Examples of functional areas include a nucleic acid extraction area, a nucleic acid purification area, a nucleic acid preparation area, a nucleic acid hybridization area, a nucleic acid amplification area, a nucleic acid detection area, a nucleic acid analysis area, and a waste area. In some embodiments, the detection area is a biochip.

The central distribution hub can be connected to a pump and a plurality of valves, and the central distribution hub can pump and inject fluids from one functional area to another. Thus, the central distribution hub makes it possible to use only one simple fluid displacement system (typically a pumping system) for most of the fluid movements of the microfluidic cartridge, for inducing depressurization and pressurization in order to displace the fluid from a functional area to another one, and to reduce the volume of the microfluidic cartridge.

Each microchannel may include a central distribution hub end ("the hub end") and a functional area end ("the area end"). The area end of a microchannel is adjacent to the corresponding functional area and, the hub end is adjacent to the central distribution hub. Each microchannel may also include a valve located near the associated area end. Thus, the microfluidic cartridge includes a plurality of valves located at or near the area ends of the hub-connected microchannels.

The valves may be spatially arranged in order to be independently actuated by an actuator. In some embodiments, the actuator is an external cam-driven actuator, linear-motion actuator, a rotational-motion actuator, a linear actuator, or a rotary actuator.

As used herein, a nucleic acid amplification reaction mixture may include a sample, which may include or be suspected of containing a target nucleic acid of interest. A variety of sample types and preparations may be used. In some embodiments, before the sample is added to a nucleic acid amplification mixture, it may be subjected to mechanical breakage, e.g., by bead beating or sonication, so that target nucleic acids may be more easily obtained from the sample. In some embodiments, extraction and purification of target nucleic acids is performed. Where a microfluidic cartridge is used, these steps may be performed in the microfluidic cartridge. In some embodiments, extraction and purification of target nucleic acids is performed in a sample preparation area of a microfluidic cartridge.

### D. Methods for Monitoring Fluorescence Signals from a Nucleic Acid Amplification Reaction for Abnormal Fluorescence Detection

In an aspect, a method of monitoring fluorescence signals from a nucleic acid amplification reaction for abnormal fluorescence detection includes measuring fluorescence signal from the nucleic acid amplification reaction at a plurality of time points in a time period during which the nucleic acid amplification reaction is occurring; determining a plurality of fluorescence intensities at at least a portion of the plurality of time points from the measured fluorescence signals; comparing each of the plurality of fluorescence intensities to a predetermined threshold. An abnormal fluorescence detection is detected if a fluorescence intensity is below the predetermined threshold.

In some embodiments, the nucleic acid amplification reaction is a thermocycled nucleic acid amplification reaction. In some embodiments, the thermocycled nucleic acid amplification reaction is a qPCR reaction. In some embodiments, the time period begins after commencement of thermocycling, or after the first thermocycle.

In some embodiments, the nucleic acid amplification reaction comprises a fluorophore. In some embodiments, the fluorophore is associated with an oligonucleotide probe. In some embodiments, the probe further includes a quencher. For example, a fluorophore may be provided as a fluorophore-containing nucleic acid (e.g., labeled detection probe, such as a TaqMan^{®} probe or a molecular torch). In some embodiments, the fluorophore-containing nucleic acid reaction mixture is a reaction mixture that is subjected to thermocycling. In some embodiments, the fluorophore-containing nucleic acid reaction mixture is a PCR reaction mixture or an RT-PCR reaction mixture.

In some embodiments, the nucleic acid amplification reaction does not comprise a fluorophore associated with an oligonucleotide and fluorescence signal comprising inherent fluorescence from one or more reagents or templates in the nucleic acid amplification reaction is measured. In some embodiments, the one or more reagents include dNTPs and/or one or more primers.

### 1. Fluorescence Measurements

The methods include measuring fluorescence signal from the nucleic acid amplification reaction at a plurality of time points in a time period during which the nucleic acid amplification reaction is occurring.

In some embodiments, the fluorescence signal is measured by a fluorescence detector. In some embodiments, the fluorescence detector is a sensor configured to measure fluorescence. In some embodiments, the fluorescence detector includes a photomultiplier or photodiode.

In some embodiments, fluorescence signals are measured from a plurality of nucleic acid amplification reactions.

### 2. Determining Fluorescence Intensities

The methods include determining a plurality of fluorescence intensities at at least a portion of the plurality of time points from the measured fluorescence signals. In some embodiments, the plurality of fluorescence intensities are measured in relative fluorescence units.

In some embodiments, the plurality of fluorescence intensities are determined by smoothing the fluorescence signals measured at the plurality of time points. Examples of smoothing include a moving average/median, local regression (loess), a low pass filter, a Savitzky-Golay smoothing filter, and a Ramer-Douglas-Peucker algorithm. Using smoothing to determine fluorescence intensities can provide more accurate detection of abnormal fluorescence detection in that a single outlier measurement that does not relate to failure of equipment such as a light source or detector is less likely to result in a below-threshold intensity. In some embodiments, smoothing the fluorescence signals includes determining a fluorescence intensity using a window of 3-21 measured fluorescence signals, such as 3, 5, 7, 9, 11, 13, 15, 17, 19, or 21 measured fluorescence signals.

In some embodiments, the plurality of fluorescence intensities are determined as a moving average or moving median of the fluorescence signals measured at the plurality of time points.

In some embodiments, determining a plurality of fluorescence intensities at at least a portion of the plurality of time points from the measured fluorescence signals includes determining a plurality of fluorescence intensities at at least a portion of the plurality of time points from the measured fluorescence signals for each of the plurality of nucleic acid amplification reactions.

### E. Detection of Abnormalities

The methods include comparing each of the plurality of fluorescence intensities to a predetermined threshold, wherein abnormal fluorescence detection is detected if a fluorescence intensity is below the predetermined threshold.

In some embodiments, a predetermined threshold may be established from reference optical runs. Reference optical runs of nucleic acid amplification reactions may include a plurality of successful and unsuccessful optical events during a method described herein. The reference runs can include optical events that were performed under different conditions. These conditions may include the presence or absence of a sample, the presence or absence of a fluorophore, the presence or absence of a LED light, and/or a functional fluorescence detector. Fluorescence measurements taken at various points during these reference optical runs may be obtained. Distributions of these measurements may be used to determine a threshold of acceptable values. For example, a threshold can be selected at a value between (i) fluorescence intensities observed in the absence of a working LED light and/or the absence of a functional fluorescence detector and (ii) fluorescence intensities observed in the presence of a working LED light and a functional fluorescence detector but in the presence or absence of sample, the correct amount of mastermix, etc. In some embodiments, the threshold is referred to as a "predetermined threshold" and is used for comparison to fluorescence intensities in the methods described herein.

Fluorescence intensities that do not satisfy a predetermined threshold may be indicative of abnormal fluorescence detection.

In some embodiments, where fluorescence intensities are determined for a plurality of nucleic acid amplification reactions, comparing each of the plurality of fluorescence intensities to a predetermined threshold includes comparing each of the plurality of fluorescence intensities to a predetermined threshold for each of the pluralities of fluorescence intensities corresponding to the plurality of nucleic acid amplification reactions.

In some embodiments, a fluorescence intensity is below the predetermined . threshold and abnormal fluorescence detection is detected. In some embodiments, the method includes discontinuing the nucleic acid amplification reaction after detecting the abnormal fluorescence detection. In some embodiments, the method includes generating an alarm after detecting the abnormal fluorescence detection. In some embodiments, the method includes notifying a user of abnormal fluorescence detection after detecting the abnormal fluorescence detection. In some embodiments, a defect in the fluorescence detector is detected if a fluorescence intensity is below the predetermined threshold.

In some embodiments, each of the plurality of fluorescence intensities are above the predetermined threshold and no abnormal fluorescence detection is detected.

### F. Nucleic Acid Amplification Systems and Computer-Readable Media

Disclosed herein are nucleic acid amplification systems. A nucleic acid amplification system refers to a device or an apparatus that may be used to perform, monitor and/or analyze fluorescent signal in nucleic acid amplification reaction as described herein. Provided herein is a nucleic acid amplification system including: (1) a docking station configured to receive a vessel comprising at least one nucleic acid amplification reaction chamber; (2) a fluorescence detector configured to measure fluorescence from a nucleic acid amplification reaction in the nucleic acid reaction chamber; and (3) a processor operably linked to the fluorescence detector and a memory. The memory includes instructions that when executed by the processor cause the nucleic acid amplification system to perform a method of monitoring fluorescence signals from a nucleic acid amplification reaction for abnormal fluorescence detection, the method comprising: a) measuring fluorescence signal from the nucleic acid amplification reaction at a plurality of time points in a time period during which the nucleic acid amplification reaction is occurring; b) determining a plurality of fluorescence intensities at at least a portion of the plurality of time points from the measured fluorescence signals; c) comparing each of the plurality of fluorescence intensities to a predetermined threshold, wherein abnormal fluorescence detection is detected if a fluorescence intensity is below the predetermined threshold.

In some embodiments, the nucleic acid amplification system includes instructions includes instructions that when executed by the processor cause the nucleic acid amplification system to perform methods of monitoring fluorescence signals from a nucleic acid amplification reaction for abnormal fluorescence detection as described herein.

In some embodiments, the nucleic acid amplification system includes a docking station to receive one or more vessels. In some embodiments, the vessel is a multi-chambered receptacle or the nucleic acid amplification chamber is contained within a multi-chambered receptacle.

In some embodiments, the nucleic acid amplification system includes a temperature controller and is capable of providing or transferring heat to nucleic acid amplification reaction chambers via one or more heating elements. In some embodiments, the nucleic acid amplification system is programmable such that it can hold nucleic acid amplification reaction chambers at set temperatures for different lengths of time.

In some embodiments, the nucleic acid amplification system includes a fluorescence detector configured to measure and record the fluorescence of one or more fluorophores in the nucleic acid amplification reaction chamber. When a vessel is present in the system, e.g., in a docking station, the fluorescence detector is disposed in proximity to a position occupied by or to be occupied by a nucleic acid amplification reaction mixture (i.e., a functional area of the vessel in optical communication with the fluorescence detector).

In some embodiments, the nucleic acid amplification system may also include sensors to monitor and/or estimate liquid handling of the nucleic acid amplification reaction chambers. In some embodiments, a sensor may be a thermal sensor, a capacitive sensor, or an infrared sensor. The nucleic acid amplification system may include a processor operably linked to the fluorescence detector and a memory. The memory includes instructions that when executed by the processor, can cause the system to perform methods of monitoring fluorescence signals from a nucleic acid amplification reaction for abnormal fluorescence detection described herein.

In some embodiments, in the event that an abnormality is detected, the nucleic acid amplification system provides one or more alarms. Alarms may be provided to indicate liquid filling abnormalities. Examples of alarms include visual notifications and audio notifications. In some examples, when an alarm is provided, the nucleic acid amplification system pauses a method of nucleic acid amplification, which may include reducing the temperature of the heating element, e.g., to about 4°C, and holding the reduced temperature for a period of time.

In some embodiments, the system is configured to discontinue the nucleic acid amplification reaction if abnormal fluorescence detection is detected.

Also disclosed herein are computer-readable media. In some embodiments, a computer-readable medium includes instructions that when executed by a processor of a nucleic acid amplification system, cause the nucleic acid amplification system to perform a method of monitoring fluorescence signals from a nucleic acid amplification reaction for abnormal fluorescence detection described herein, e.g., including the following steps: measuring fluorescence signal from the nucleic acid amplification reaction at a plurality of time points in a time period during which the nucleic acid amplification reaction is occurring; determining a plurality of fluorescence intensities at at least a portion of the plurality of time points from the measured fluorescence signals; comparing each of the plurality of fluorescence intensities to a predetermined threshold, wherein abnormal fluorescence detection is detected if a fluorescence intensity is below the predetermined threshold.

In some embodiments, the computer-readable medium includes instructions for performing methods of monitoring fluorescence signals from a nucleic acid amplification reaction for abnormal fluorescence detection as described herein. In some embodiments, these instructions are executed by a processor of the nucleic acid amplification system as described herein.

In some embodiments, the method includes discontinuing a nucleic acid amplification reaction if an abnormality is detected.

### EXAMPLES

### Example 1

This Example describes performing reference optical runs of nucleic acid amplification reactions under various conditions including normal conditions, conditions with fluorescence detection abnormalities, and conditions with other abnormalities. This collection of reference runs provided a basis for predetermining a threshold to compare to fluorescence intensities in the methods described herein.

Reaction mixtures including Amplidiag^{®} Multiplex PCR Master Mix; a nucleic acid template; forward and reverse primers; and fluorescent probes were prepared.

The reaction mixtures were subjected to amplification in a Novodiag thermal cycler. Fluorescence measurements from the nucleic acid amplification reaction chamber were obtained at various points during these reference optical runs, including before and after the LED light source was turned off. (FIG. 1).

Additional runs were performed and fluorescence intensities were obtained in at a plurality of timepoints during various scenarios (e.g., LED light turned off, no sample, less reagents, etc.). (FIG. 2). Values in a certain range, or that satisfy or do not satisfy a certain threshold are indicative of one or more optical errors or equipment failures. As noted in Figure 2, a threshold of 100 RFUs in the blue channel was used as a threshold to indicate an abnormal fluorescence detection (in this case, no LED light). Thus, the method was effective at detecting abnormal fluorescence detection and distinguishing from normal fluorescence detection (from both normal and abnormal reactions) based on fluorescence intensities obtained from nucleic acid amplification reactions.

### EQUIVALENTS

The foregoing written specification is considered to be sufficient to enable one skilled in the art to practice the embodiments. The foregoing description and Examples detail certain embodiments and describes the best mode contemplated by the inventors. It will be appreciated, however, that no matter how detailed the foregoing may appear in text, the embodiment may be practiced in many ways and should be construed in accordance with the appended claims and any equivalents thereof.

## Claims

1. A method of monitoring fluorescence signals from a nucleic acid amplification reaction for abnormal fluorescence detection, the method comprising
a) measuring fluorescence signal from the nucleic acid amplification reaction at a plurality of time points in a time period during which the nucleic acid amplification reaction is occurring;
b) determining a plurality of fluorescence intensities at at least a portion of the plurality of time points from the measured fluorescence signals;
c) comparing each of the plurality of fluorescence intensities to a predetermined threshold, wherein abnormal fluorescence detection is detected if a fluorescence intensity is below the predetermined threshold.

2. The method of claim 1, wherein the nucleic acid amplification reaction is a thermocycled nucleic acid amplification reaction, optionally wherein the time period begins after commencement of thermocycling, or after the first thermocycle.

3. The method of claim 1 or 2, wherein the plurality of fluorescence intensities are determined by smoothing the fluorescence signals measured at the plurality of time points, and/or a moving average or moving median of the fluorescence signals measured at the plurality of time points.

4. The method of any one of the preceding claims, wherein the fluorescence signal is measured by a fluorescence detector, optionally wherein the fluorescence detector comprises a photomultiplier or photodiode.

5. The method of claim 4, wherein a defect in the fluorescence detector is detected if a fluorescence intensity is below the predetermined threshold.

6. The method of any one of the preceding claims, wherein fluorescence signals are measured from a plurality of nucleic acid amplification reactions in step a), optionally wherein the plurality of fluorophore-containing nucleic acid amplification reactions is contained in a multi-well plate or in a plurality of tubes.

7. The method of claim 6, wherein step b) comprises determining a plurality of fluorescence intensities at at least a portion of the plurality of time points from the measured fluorescence signals for each of the plurality of nucleic acid amplification reactions, and optionally wherein step c) comprises comparing each of the plurality of fluorescence intensities to a predetermined threshold for each of the pluralities of fluorescence intensities corresponding to the plurality of nucleic acid amplification reactions.

8. The method of any one of the preceding claims, wherein the nucleic acid amplification reaction comprises a fluorophore, optionally wherein the fluorophore is associated with an oligonucleotide probe, optionally wherein the oligonucleotide probe further comprises a quencher; or wherein the nucleic acid amplification reaction does not comprise a fluorophore associated with an oligonucleotide and fluorescence signal comprising inherent fluorescence from one or more reagents or templates in the nucleic acid amplification reaction is measured, optionally wherein the one or more reagents comprise dNTPs and/or one or more primers.

9. The method of any one of the preceding claims, wherein a fluorescence intensity is below the predetermined threshold and abnormal fluorescence detection is detected, optionally wherein the method further comprises discontinuing the nucleic acid amplification reaction after detecting the abnormal fluorescence detection; or wherein each of the plurality of fluorescence intensities are above the predetermined threshold and no abnormal fluorescence detection is detected.

10. The method of any one of the preceding, wherein the method further comprises discontinuing a nucleic acid amplification reaction if an abnormality is detected.

11. A nucleic acid amplification system comprising:
a docking station configured to receive a vessel comprising at least one nucleic acid amplification reaction chamber;
a fluorescence detector configured to measure fluorescence from a nucleic acid amplification reaction in the nucleic acid reaction chamber; and
a processor operably linked to the fluorescence detector and a memory;
the memory comprising instructions that when executed by the processor cause the nucleic acid amplification system to perform a method of monitoring fluorescence signals from a nucleic acid amplification reaction for abnormal fluorescence detection, the method comprising:
a) measuring fluorescence signal from the nucleic acid amplification reaction at a plurality of time points in a time period during which the nucleic acid amplification reaction is occurring;
b) determining a plurality of fluorescence intensities at at least a portion of the plurality of time points from the measured fluorescence signals;
c) comparing each of the plurality of fluorescence intensities to a predetermined threshold, wherein abnormal fluorescence detection is detected if a fluorescence intensity is below the predetermined threshold.

12. The system of claim 11, wherein the nucleic acid amplification reaction is contained within a microfluidic cartridge.

13. The system of claim 12, wherein the microfluidic cartridge comprises:
a) a plurality of functional areas comprising a sample preparation area, a nucleic acid amplification area, and a waste area;
b) a central distribution hub; and
c) a pump, a plurality of valves, and a fluidic network of microchannels connecting the functional areas to the hub;
wherein the pump, plurality of valves, and fluidic network of microchannels can drive movement of a fluid from a first functional area through the central distribution hub to a second functional area of the plurality of functional areas;
and the nucleic acid amplification reaction is within the nucleic acid amplification area.

14. The system of any one of claims 11-13, wherein the system is configured to discontinue the nucleic acid amplification reaction if abnormal fluorescence detection is detected.

15. The system of any one of claims 11-14, wherein the memory comprises instructions that when executed by the processor cause the nucleic acid amplification system to perform the method of any of claims 1-10.

16. A computer-readable medium comprising:
instructions that when executed by a processor of a nucleic acid amplification system cause the nucleic acid amplification system to perform a method of monitoring fluorescence signals from a nucleic acid amplification reaction for abnormal fluorescence detection, the method comprising:
a) measuring fluorescence signal from the nucleic acid amplification reaction at a plurality of time points in a time period during which the nucleic acid amplification reaction is occurring;
b) determining a plurality of fluorescence intensities at at least a portion of the plurality of time points from the measured fluorescence signals;
c) comparing each of the plurality of fluorescence intensities to a predetermined threshold, wherein abnormal fluorescence detection is detected if a fluorescence intensity is below the predetermined threshold.

17. The computer-readable medium of claim 16, comprises instructions that when executed by a processor of a nucleic acid amplification system cause the nucleic acid amplification system to perform the method of claims 1-10.
